(19) Europäisches Patentamt
European Patent Office
Office européen des brevets

(11) Publication number: **0 006 357**
**A1**

(12) **EUROPEAN PATENT APPLICATION**

(21) Application number: **79301158.6**

(22) Date of filing: **15.06.79**

(51) Int. Cl.³: **C 07 G 7/00**, G 01 N 33/48

(30) Priority: **16.06.78 JP 72895/78**
**16.06.78 JP 72896/78**

(43) Date of publication of application: **09.01.80**
**Bulletin 80/1**

(84) Designated Contracting States: **DE FR GB NL**

(71) Applicant: **SANKYO COMPANY LIMITED, No. 1-6, 3-chome Nihonbashi Honcho Chuo-ku, Tokyo (JP)**

(72) Inventor: **Oh-Uti, Kiyota, 4-5, 3-chome, Shigemorishin-cho, Hirosaki-city Aomori-prefecture (JP)**

(74) Representative: **Gibson, Christian John Robert, Marks & Clerk 57/60 Lincoln's Inn Fields, London WC2A 3LS (GB)**

(54) **Composition and method for detecting gastric cancer.**

(57) A physiologically active substance, which I call «AIF» (Anaemia Inducing Factor), has been isolated from the human placenta and from the body fluids and tissues of patients suffering from gastric cancers and has been implicated in the anaemia often associated with malignant tumours, particularly gastric cancers. The purified AIF forms the basis for an antigen-antibody test for the presence of gastric cancer, using a calibration curve such as that shown in Figure 3.

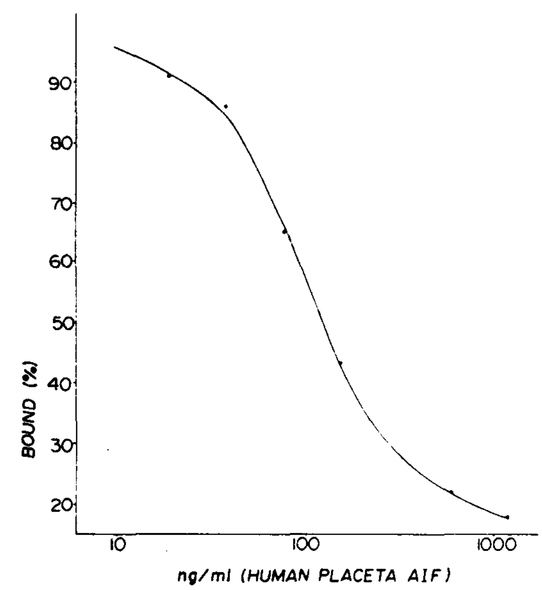


EP 0 006 357 A1

"COMPOSITION AND METHOD FOR DETECTING GASTRIC CANCER"

The invention relates to a new physiologically active substance which I call "AIF" (Anaemia Inducing Factor) and to the use of this substance to test for the presence of gastric cancer.

Many cancers can be treated, provided that they are detected and treatment begun at a sufficiently early stage. In many cases, however, the cancers produce symptoms which are not readily detectable and/or which can easily be confused with symptoms of other diseases. There is, therefore, a need for a relatively easy means of detecting the presence of various types of cancer in the human body.

Anaemia has frequently been reported in patients suffering from malignant tumours, particularly patients attacked by gastric cancers ["Gastric Cancer in Surgery" (in Japanese), Masao Muto, 1st edition, page 230, Kinbara Publishing Co. Limited, Tokyo (1963)]. It has also been reported than an anaemia-inducing activity can be detected in gastric juices, gastric cancer tissues, serum and urine taken from patients suffering from gastric cancers; however, the entity inducing anaemia has not hitherto been isolated or described.

In "Hirosaki Igaku", by Kiyota Oh-Uti and Taisuke Nishikawa, Volume 27, pages 379 - 396 (1975), I have previously reported that an entity inducing anaemia is present not only in gastric cancer tissues and gastric juices, serum and urine derived from patients suffering from gastric cancers, but also in extracts of human placenta. However, in my previous work, I have only prepared crude extracts of mixtures containing the active substance and have not succeeded in separating and identifying the substance itself.

I have now isolated the substance, AIF, which induces anaemia in patients suffering from gastric cancer and have determined that this substance is a single compound.

The new physiologically active substance, AIF, provided by the present invention is characterized by the following properties:

1. Elemental Analysis

C, 42.50 - 46.60%; H, 6.30 - 6.55%; N, 6.38 - 7.51%.

2. Standard Sedimentation Coefficient

1.74S(Svenberg) = S20W.

3. Standard Diffusion Coefficient

$8.28 \times 10^{-7} cm^2/sec.$

4. Molecular Weight

19,230 (calculated from standard sedimentation coefficient and standard diffusion coefficient);

20,137 (calculated from amino acid analysis);

21,000 (calculated from sodium dodecyl sulphate-polyacrylamide electrophoresis).

5. Isoelectric Point

pH = 4.58.

6. Ultraviolet Absorption Spectrum

As shown in Figure 1, measured in a 1% w/v sodium chloride solution at 0.358 mg/ml;

$\varepsilon_{278} = 15.6 \times 10^3$.

7. Amino Acid Composition

As shown in Table 1:

Table 1

| Amino acid | Analysis mole/mole | Number of residues per molecule as an integer |
|---|---|---|
| Lysine | 5.62 - 5.81 | 6 |
| Histidine | 0.90 - 1.12 | 1 |
| Arginine | 1.93 - 2.01 | 2 |
| Aspartic acid | 13.68 - 14.22 | 14 |
| Threonine | 5.62 - 5.87 | 6 |
| Serine | 1.79 - 2.22 | 2 |
| Glutamic acid | 8.31 - 9.17 | 9 |
| Proline | 2.19 - 2.38 | 2 |
| Glycine | 1.72 - 2.48 | 2 |
| Alanine | 3.35 - 4.03 | 4 |
| Cysteine | 7.55 - 8.25 | 8 |
| Valine | 3.84 - 4.02 | 4 |
| Methionine | 0 - 0.14 | 0 |

Cont..

Table 1 (continued)

| Amino acid | Analysis mole/mole | Number of residues per molecule as an integer |
|---|---|---|
| Isoleucine | 1.08 - 1.09 | 1 |
| Leucine | 6.48 - 6.76 | 7 |
| Tyrosine | 3.57 - 3.73 | 4 |
| Phenylalanine | 3.62 - 3.71 | 4 |
| Tryptophane | 1.90 - 2.25 | 2 |

Analysis was effected after hydrolysis with 6N hydrochloric acid at 110°C for 24 hours and the number of amino acid residues was calculated on the basis of a molecular weight of 19,230 and a sugar content of 55.6% by weight.

8. Sugar composition

Sugar components constitute 51 - 58% by weight of the molecule, when measured by the phenol-sulphuric acid method using glucose as a standard, glucosamine constitutes 2.08 - 2.21% by weight (by amino acid analysis) and glucose itself constitutes 30.8 - 34.6% by weight (by gas chromatography).

9. Anaemia Inducing Activity

Reduces the number of erythrocytes by not less than 8.9% 2 hours after intravenous administration to rabbits at a dose of 2.7 µg/kg body weight.

The discovery and isolation of substantially pure AIF in accordance with the invention has also provided a method of dectecting cancer-related anaemia (and hence the presence of gastric cancer) by detecting AIF in human body fluids.

Thus the invention further provides a method of detecting cancer-related anaemia, which comprises the *in vitro* immunochemical determination of the presence in body fluids of AIF using an anti-AIF antibody.

In the accompanying drawings:

Figure 1 shows the ultraviolet absorption spectrum of AIF, measured in a 1% w/v aqueous sodium chloride solution at 0.358 mg/ml;

Figure 2 shows AIF concentrations in various human sera;

Figure 3 shows a calibration curve for determining AIF concentration in a fluid of unknown concentration, the curve being prepared from results obtained by a double antibody technique using the reaction between human placental AIF and AIF antibody; and

Figure 4 is a calibration curve similar to

Figure 3 but prepared using AIF from a gastric cancer patient.

Although AIF is present in, and can thus be isolated from, many of the tissues and body fluids of gastric cancer patients (e.g. gastric cancer tissues, gastric juices, urine and serum of such patients), I have surprisingly found that it is also present in all human placenta, whether or not the donor is suffering from gastric cancer. Accordingly, in view of the wide availability of placenta as raw materials, these are the preferred materials for the extraction of AIF. Whichever raw material is chosen, the AIF may be separated first as a crude sample containing the desired activity and then as a pure compound. I have found that a protein-chemically single AIF may be isolated by purification through various combinations of ion exchange chromatography, gel filtration (with, for example, Sephadex or Bio-gel), electrophoresis, precipitation with an organic solvent (e.g. methanol, ethanol or acetone) or dialysis. By an appropriate combination of such steps, it is possible to obtain AIF at a purity some 1,000 times as high as that of the crude sample (according to an erythrocyte haemolytic test).

On subjecting the resulting AIF sample to electrophoresis on a polyacrylamide disc, colouring the protein portion with amido black and the sugar portion with periodic acid Schiff's reagent, the colour bands are observed at the same position, indicating that the compound is a single glycoprotein. The physical and chemical properties of the compound have already been described. The sugar content of the compound may vary from 51 to 58% by weight, with a mean of 55.6% by weight by the phenol-sulphuric acid method using glucose as a standard.

AIF has the physiological effect of destroying erythrocytes and this effect can be used as the basis for assessing its purity or for assessing the concentration of AIF in a sample of body fluids. For example, AIF administered intravenously to rabbits at a dose of 2.3 μg/kg body weight can reduce the number of erythrocytes by 8.9 - 10%, or even more, 2 hours after administration, as determined by blood samples collected at that time. Also, if AIF is added in an amount of 0.3 μg/ml to rabbit blood which has been treated to prevent blood coagulation and the sample is left at room temperature for several hours to precipitate erythrocytes, a noticeable red colour can be seen in the supernatant, which clearly demonstrates the occurrence of a haemolytic reaction.

An anti-AIF serum was prepared from rabbits sensitized with AIF and tested immunologically; it showed response neither to α-fetoprotein nor to carcino-embryonic antigen which is already known as a specific cancer antigen, nor did it show any response to HBs antigen, which is known as an antibody to hepatitis. Agar electrophoresis of carcino-embryonic protein antigen generally shows migration patterns in the region of $\beta$-$\alpha_2$ globulin, whereas AIF shows migration patterns in the region of γ-globulin. In view of these properties, it is concluded that AIF is a new substance with a novel antigenicity.

In the past, the cancer-related anaemia-inducing activity of body fluids or tissues has been determined by erythrocyte osmotic pressure resistance tests, erythrocyte counts with rabbits, erythrocyte haemolytic tests and immunoelectrophoresis; however, it is difficult to demonstrate the presence of such activity in human serum or other body fluids having a low anaemia-inducing activity. Accordingly, this activity could not previously have been determined except by concentration or partial purification of the body tissues or fluids concerned and this is not practical in the course of routine clinical tests. However, the isolation of pure AIF in accordance with the present invention makes possible an immunochemical determination method for clinical diagnosis which can determine AIF concentration by direct use of a body fluid. By using AIF (which need not be purified) as an antigen to provoke the production of an AIF antibody in a suitable mammal, for example rabbits, and then producing a calibration curve for the reaction of purified AIF at known concentrations with the resulting antibody, the curve obtained may then be used to determine, by a similar reaction with AIF antibody, the AIF concentration of the body fluid to be tested.

The progress of the reaction between the AIF and its antibody can be determined by any method applicable to this type of reaction, for example radio-immunoassay, enzyme-immunoassay, immunoassay with fluorescent-labelled antigen or antibody, laser immunoassay, erythrocyte or latex agglutination assay, or single radial immunodiffusion assay.

A preferred procedure comprises injecting AIF subcutaneously into an animal other than a human being (for example a rabbit) to cause the animal to produce AIF antibody, which is then extracted. Then, purified AIF is labelled with a radioactive substance, for example $^{125}I$ (in place of radioactive labelling, fluorescent labelling, enzyme labelling or labelling with a spin compound or a luminous substance may be employed), and then a calibration curve for AIF is drawn up by the antigen (AIF)-antibody reaction with the antibody produced as described above using the labelled AIF and a standard liquid of known concentration. Subsequently, the same antigen-antibody reaction is conducted using the serum, urine or other body fluids of a patient suspected of having gastric cancer and the amount of AIF contained in that fluid can be determined from this calibration curve. Relatively low concentrations of AIF in the body fluid under test can be detected by these means.

Figure 2 of the accompanying drawings shows AIF concentrations in human serum measured as described above, from which it is clear that AIF is detected in gastric cancer patients at a significantly higher concentration than in patients suffering from other diseases and in normal patients; the level of AIF in body fluids is therefore a useful diagnostic method for gastric cancer.

Figure 3 of the accompanying drawings shows a calibration curve prepared as described above by conducting radioimmunoassay according to a double antibody technique using the reaction between human placental AIF and its antibody. It can be seen that AIF concentrations down to about 10 ng/ml are measurable.

A calibration curve has also been produced as described above from an AIF-containing sample partly purified from the serum of gastric cancer patients and this curve is shown in Figure 4 of the accompanying drawings.

Comparing Figures 3 and 4, it is apparent that human placental AIF and the AIF derived from the serum of gastric cancer patients have the same immunochemical properties and thus that AIF measurement is a useful new diagnostic method for gastric cancer.

The invention is further illustrated by the following Examples.

## EXAMPLE 1

### Preparation of purified AIF

A solution of 300 mg of a crude human placental AIF extract (obtained by known means) in 30 ml of a 0.005M phosphate buffer (pH 8.0) was dialyzed overnight in 2 litres of the same buffer. The dialyzate was adsorbed on a 2.2 x 26 cm column containing DEAE cellulose (available from Serva Co. Limited) buffered with the same buffer. This column was then eluted with the same buffer containing sodium chloride by the gradient elution method, raising the sodium chloride concentration gradually up to 0.2M to collect those fractions containing the desired active entity.

The content of the active entity in the eluted fractions was determined by an erythrocyte haemolytic test. A homogeneous solution was prepared by adding 1.5 ml of 3.8% w/v aqueous sodium citrate to 7.5 ml of fresh rabbit blood and then adding 12 ml of physiological saline thereto. 0.5 ml of this solution was poured into a small test tube and immediately thereafter 20 µl of a test liquid were added on top. The tubes were left at room temperature for 1 - 2 hours, at the end of which time the erythrocytes were precipitated and the tubes were evaluated to determine whether or not haemolysis had taken place in the supernatant.

The fractionated liquids were dialyzed overnight in 2 litres of a 0.01M acetate buffer (pH 4.5) and the dialyzate was adsorbed on a 1.2 x 15 cm column containing CM cellulose (available from Serva Co. Limited) buffered with the same buffer. This was then eluted with the same buffer by the gradient elution method, the sodium chloride concentration being gradually raised to 0.2M,and those fractions containing AIF were collected.

The collected fractions were diluted with water to four times their original volume and adsorbed on a 0.8 x 10 cm column containing CM cellulose buffered with a 0.01M acetate buffer (pH 4.5). This was then washed with the same acetate buffer in an amount 20 times the volume of the column and subsequently eluted with the same buffer containing 0.15M sodium chloride. The active fractions obtained were then concentrated and the concentrated AIF solution was subjected to gel filtration using a 2.2 x 132 cm column containing Sephadex G-75 (available from Pharmacia Co. Limited) buffered with a 0.02M phosphate buffer solution (pH 7.0) containing 0.1M sodium chloride. There were obtained 1.2 mg of purified human placental AIF having the properties heretofore described.

EXAMPLE 2

Preparation of AIF antibody

Crude human placental AIF samples were partly purified by column chromatography using DEAE cellulose. To a solution of 2 mg of the purified sample in 1 ml of physiological saline was added an equal volume of complete Freund's adjuvant. The resulting emulsified liquid was subcutaneously injected into the backs of rabbits (body weight about 3 kg) in three or four different positions on each rabbit. After 2 weeks, 1 ml of an AIF emulsion prepared in the same manner as described above was administered as a booster dose and a further booster dose of 1 ml of the same AIF emulsion was given after a further 2 weeks. 1 week after the final injection, blood was collected from the rabbits and the serum separated therefrom. The serum was inactivated by heating at 56°C for 30 minutes. This serum contained anti-AIF antibodies.

## EXAMPLE 3

### Labelling AIF

A solution of 100 µg of purified human placental AIF (prepared as described in Example 1) in 0.1 ml of a 0.05M phosphate buffer (pH 7.0) was mixed with a solution of 1 mCi of $Na^{125}I$ in 0.1 ml of the same buffer. 0.25 ml of a 3 mg/ml aqueous solution of chloramine T were then added thereto and the mixture was allowed to react under ice-cooling for 30 seconds. Immediately thereafter, 0.1 ml of a 25 mg/ml aqueous solution of sodium metabisulphite was added and the mixture was stirred for 5 minutes. 0.45 ml of a 10% w/v aqueous potassium iodide solution was added and the mixture was then subjected to gel filtration through a 1.0 x 20 cm column containing Sephadex G-25 buffered with a 0.05M borate buffer (pH 8.6) containing 1% bovine serum albumin. This separated labelled AIF and unreacted reagents, giving the labelled AIF with an activity of 1.45 µCi/µg AIF.

## EXAMPLE 4

### Preparation of a calibration curve

A calibration curve was determined using standard solutions of known concentration containing a mixture of purified human placental AIF (obtained as described in Example 1) and the $^{125}I$-labelled AIF obtained in Example 3. 50 μl of a standard solution having a known AIF concentration were mixed with 50 μl of labelled AIF solution (15,000 cpm/50 μl, prepared with 0.05M borate buffer of pH 8.6 containing 1.2% normal rabbit serum). 50 μl of a first antibody solution (prepared by a 500-fold dilution of the anti-AIF rabbit serum prepared in Example 2 with a 0.05M borate buffer of pH 8.6 containing 1% bovine serum albumin) were added thereto and the mixture was allowed to react at 4°C for 24 hours. At the end of this time, 50 μl of a second antibody solution (prepared by 10-fold dilution of anti-rabbit γ-globulin goat serum with the same buffer) were added and the mixture allowed to react at 4°C for 24 hours. The reaction mixture was then centrifuged at 3000 rpm for 15 minutes and the radioactivity of the combined precipitates was measured. This was carried out over a large range of AIF concentrations and the results were plotted as a calibration curve. This curve is shown in Figure 3 of the accompanying drawings. Using this calibration curve, an AIF concentration can be determined with great precision over the range 10 - 600 ng/ml.

## EXAMPLE 5

### AIF determination of gastric cancer patient serum

Test samples were prepared by serial dilution of partly purified AIF obtained from the serum of gastric cancer patients. These samples were measured by the procedures described in Example 4 and the resulting measurements were used to produce the calibration curve shown in Figure 4 of the accompanying drawings. It can be seen that the AIF in gastric cancer patients serum is measurable using labelled human placental AIF and anti-human placental AIF serum as reagents.

## EXAMPLE 6

### AIF determination in serum

The AIF content of the sera of normal humans and of gastric cancer patients was determined by the double antibody technique described in Example 4. The results are given in Table 2.

Table 2

| Test sample | No. of test samples | AIF concentration |
|---|---|---|
| Normal human serum | 28 | 10 - 90 ng/ml |
| Gastric cancer patient serum | 114: 37 | < 90 ng/ml |
| | 114: 77 | >90 ng/ml |

CLAIMS:

1. An Anaemia Inducing Factor characterized by the following physical and chemical properties:

(i) Elemental analysis
C, 42.50 - 46.60%; H, 6.30 - 6.55%; N, 6.38 - 7.51%;

(ii) Standard sedimentation coefficient
1.74S(Svenberg) = S20W;

(iii) Standard diffusion coefficient
$8.28 \times 10^{-7} cm^2/sec$;

(iv) Molecular weight
19,230 (calculated from standard sedimentation coefficient and standard diffusion coefficient);

20,137 (calculated from amino acid analysis);

21,000 (calculated from sodium dodecyl sulphate-polyacrylamide electrophoresis);

(v) Isoelectric point
pH = 4.58;

(vi) Ultraviolet absorption spectrum
As shown in Figure 1, measured in a 1% w/v sodium chloride solution at 0.35 mg/ml;

$\varepsilon_{278} = 15.6 \times 10^3$,

(vii) Amino acid composition

As shown in Table 1:

Table 1

| Amino acid | Analysis mole/mole | Number of residues per molecule as an integer |
|---|---|---|
| Lysine | 5.62 - 5.81 | 6 |
| Histidine | 0.90 - 1.12 | 1 |
| Arginine | 1.93 - 2.01 | 2 |
| Aspartic acid | 13.68 - 14.22 | 14 |
| Threonine | 5.62 - 5.87 | 6 |
| Serine | 1.79 - 2.22 | 2 |
| Glutamic acid | 8.31 - 9.17 | 9 |
| Proline | 2.19 - 2.38 | 2 |
| Glycine | 1.72 - 2.48 | 2 |
| Alanine | 3.35 - 4.03 | 4 |
| Cysteine | 7.55 - 8.25 | 8 |
| Valine | 3.84 - 4.02 | 4 |
| Methionine | 0 - 0.14 | 0 |



Continued

Table 1 (continued)

| Amino acid | Analysis mole/mole | Number of residues per molecule as an integer |
| --- | --- | --- |
| Isoleucine | 1.08 - 1.09 | 1 |
| Leucine | 6.48 - 6.76 | 7 |
| Tyrosine | 3.57 - 3.73 | 4 |
| Phenylalanine | 3.62 - 3.71 | 4 |
| Tryptophane | 1.90 - 2.25 | 2 |

(analysis effected after hydrolysis with 6N hydrochloric acid at 110°C for 24 hours, the number of amino acid residues was calculated on the basis of a molecular weight of 19,230 and a sugar content of 55.6% by weight);

(viii) Sugar composition

Sugar components constitute 51 - 58% by weight of the molecule, when measured by the phenol-sulphuric acid method using glucose as a standard, glucosamine constitutes 2.08 - 2.21% by weight (by amino acid analysis) and glucose itself constitutes 30.8 - 34.6% by weight

(by gas chromatography);

(ix) Anaemia Inducing Activity
Reduces the number of erythrocytes by not less than 8.9% 2 hours after intravenous administration to rabbits at a dose of 2.7 μg/kg body weight.

2. A method of producing AIF, which comprises extracting AIF from human placenta.

3. A method of detecting cancer-related anaemia which comprises the <u>in vitro</u> immunochemical determination of the presence in body fluids of AIF using an anti-AIF antibody.

4. A method according to Claim 3, which comprises the steps:

(a) injecting AIF into an animal to produce AIF antibodies;

(b) extracting AIF antibodies from said animal;

(c) reacting said antibodies with solutions of known concentration containing purified AIF and using the results to produce a calibration curve; and

(d) reacting AIF from the body fluids or tissues under test with said AIF antibodies and determining the concentration of AIF in the tissues or fluids under test from said calibration curve.

5. A method according to Claim 4, in which the purified AIF employed in step (c) is human placental AIF.

6. A method according to Claim 4, in which the purified AIF employed in step (c) is AIF from gastric cancer patient serum.

7. A method according to any one of Claims 4 to 6, in which the reactions in steps (c) and (d) are carried out by the double antibody technique.

8. A method according to any one of Claims 4 to 7, in which said animals are rabbits.

FIG. 1

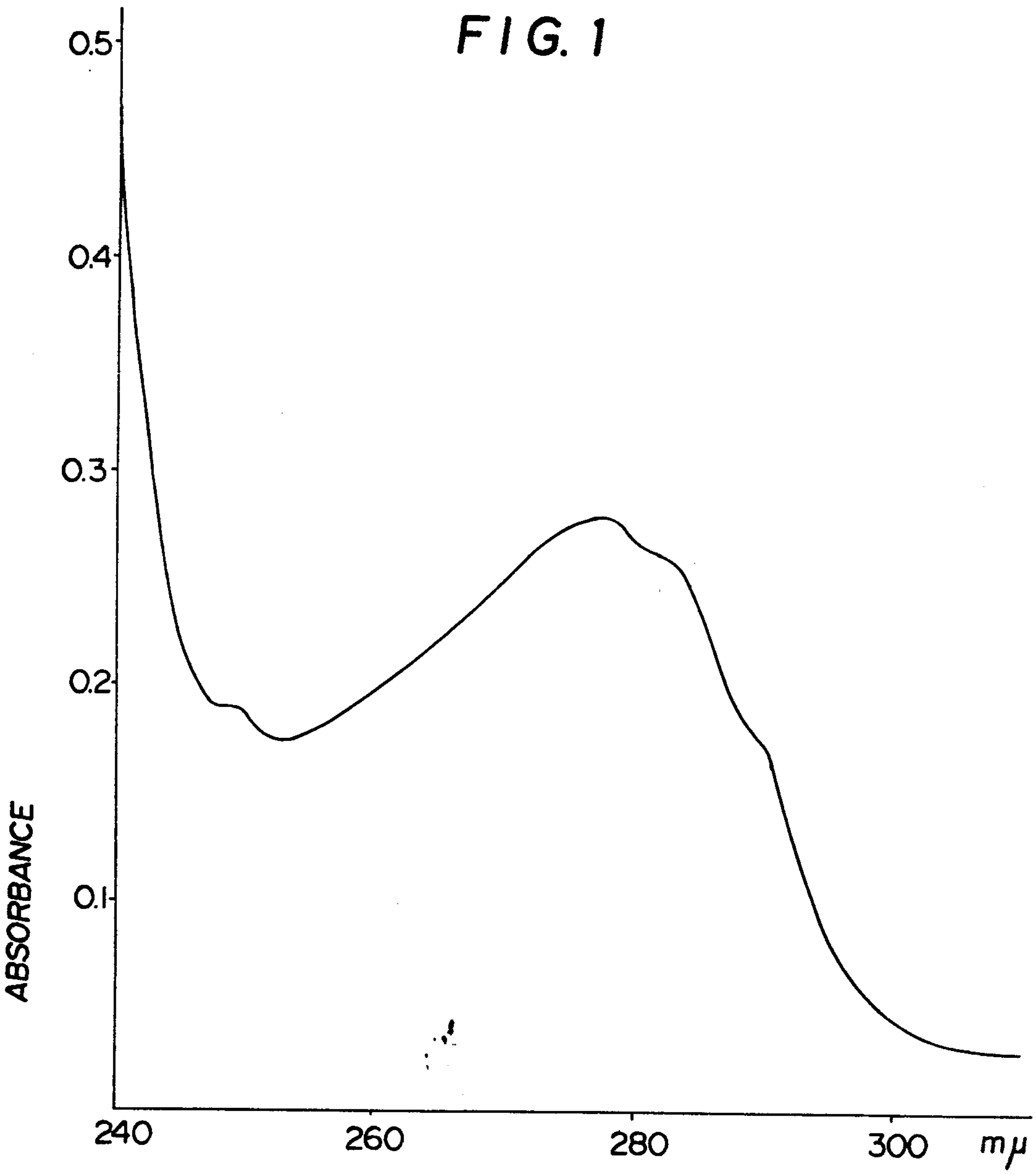

FIG. 2

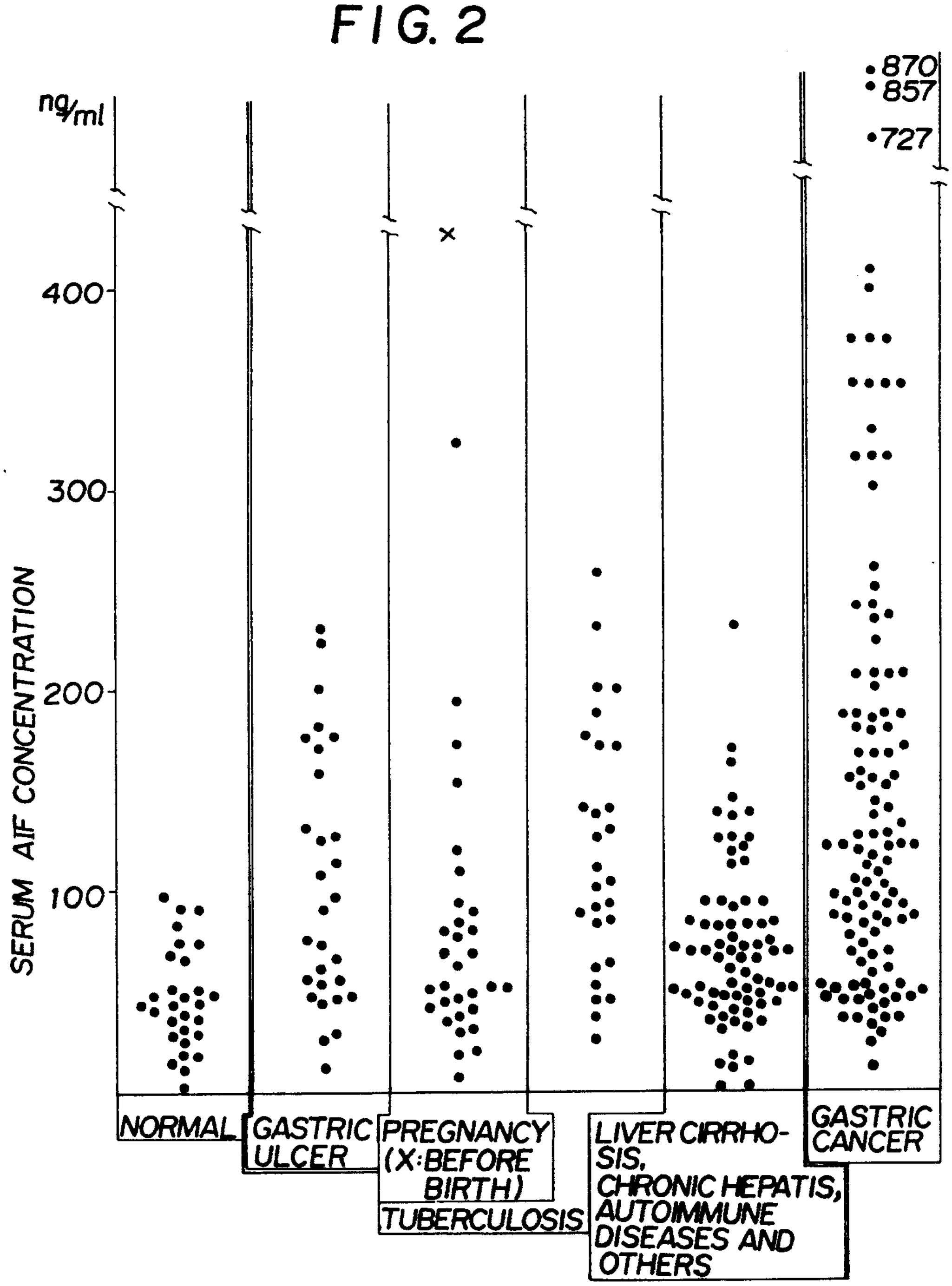
ng/ml
SERUM AIF CONCENTRATION
400
300
200
100
870
857
727
NORMAL
GASTRIC ULCER
PREGNANCY (X: BEFORE BIRTH)
TUBERCULOSIS
LIVER CIRRHOSIS, CHRONIC HEPATIS, AUTOIMMUNE DISEASES AND OTHERS
GASTRIC CANCER

FIG. 3

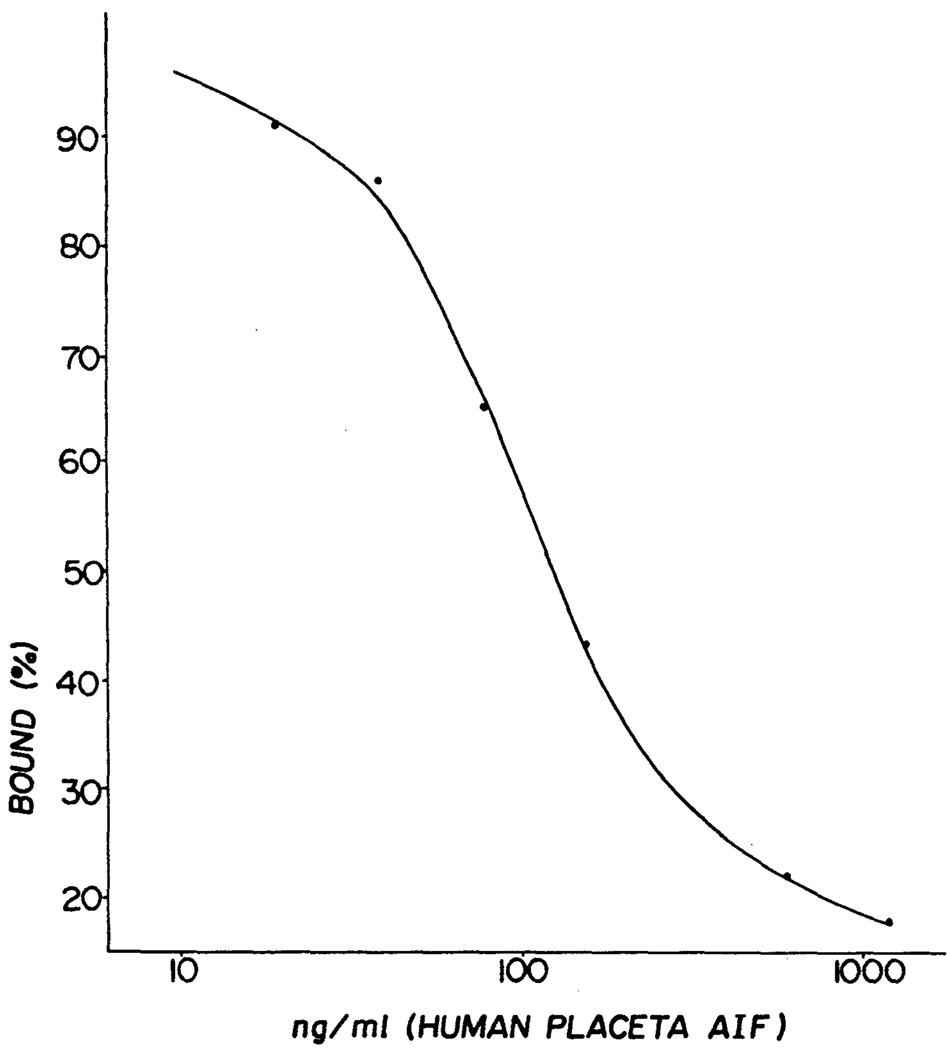

FIG. 4

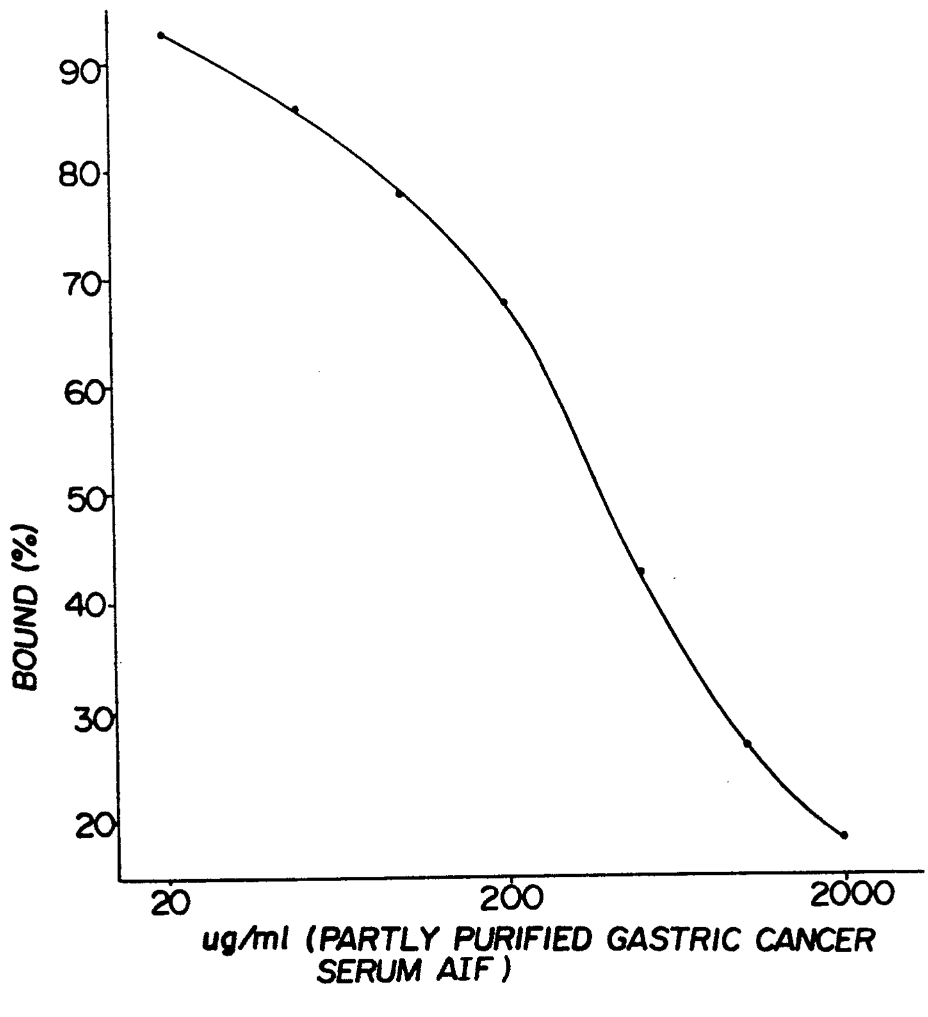
BOUND (%)
90
80
70
60
50
40
30
20
20
200
2000
ug/ml (PARTLY PURIFIED GASTRIC CANCER SERUM AIF)

0006357

European Patent Office

# EUROPEAN SEARCH REPORT

Application number

EP 79 301 158.6

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | | | C 07 G 7/00 |
| A,P | GB - A - 1 524 221 (S. BOGOCH)<br>* whole document *<br>-- | 1,3 | G 01 N 33/48 |
| A,P | GB - A - 1 532 803 (S. BOGOCH)<br>* whole document *<br>-- | 1,3 | |
| A | Chemical Abstracts, Volume 59, Nr. 13, December 23, 1963<br>(COLUMBUS,OHIO, USA)<br>Y. YONEYAMA " Extraction of a sequestration factor of blood cells from the serum of experimental rabbits"<br>column 15 678 EF<br>& Tokyo Ika Daigaku Zasshi Suppl. Volume 18, Nr. 5, 1960, pages 1861 to 1881<br>-- | | TECHNICAL FIELDS SEARCHED (Int.Cl.3)<br>C 07 G 7/00<br>G 01 N 33/48 |
| A | Chemical Abstracts, Volume 59, Nr. 13, December 23, 1963<br>(COLUMBUS, OHIO, USA)<br>T. SETSUDA et al. "An anemia-producing factor in serum and urine of patients with aplastic anemia"<br>column 15 713 H to 15 714 C<br>& Proc. Intern. Congr. Hematol., 8th, Tokyo,Volume 2, 1960, pages 1193 to 1196<br>---- | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| Berlin | 11-09-1979 | KNAACK |